# EUROPEAN PATENT APPLICATION

(11) **EP 4 066 748 A1**
(43) Date of publication of application: **05.10.2022**
(21) Application number: 21166184.8
(22) Date of filing: 31.03.2021
(51) Int. Cl.: A61B 10/02

(54) **NEEDLE ARRANGEMENT FOR BIOPSY**

(71) Applicant: Xaga Surgical AB, 256 67 Helsingborg (SE)
(72) Inventor: FORSVALL, Andreas, 256 67 HELSINGBORG (SE); UVNÄS, Krister, 247 35 SÖDRA SANDBY (SE); EDBERG, Mats, 241 37 ESLÖV (SE)
(74) Representative: Brann AB

(57) **Abstract**

A biopsy needle arrangement (20) comprises a needle sheath (22) constituting an elongated tube having a sheath opening (203) at a distal end (202). The biopsy needle arrangement (20) also comprises a needle (21) having a length that is greater than the length of the needle sheath (22). The needle (21) comprises an elongated shaft portion configured to fit inside and slide relative to the needle sheath (22), and a tip (23) located at a distal end (212) of the needle (21). The biopsy needle arrangement (20) further comprises an intermediary (25) having a length that is greater than the length of the needle sheath (22). The intermediary (25) comprises an elongated shaft portion parallel with the needle (21) and configured to fit inside the needle sheath (22) and configured to slide relative to the needle sheath (22) and slide relative to the needle (21).

## Description

### TECHNICAL FIELD

Embodiments herein relate to a biopsy needle arrangement comprising a needle inside a tubular needle sheath.

### BACKGROUND

Within the field of medicine, needle arrangements for various purposes exist, for example biopsy needle arrangements. Biopsy needle arrangements may be configured for taking biopsy samples of hard and soft tissue. Soft tissue biopsy may include taking biopsy samples from, e.g., a liver, a kidney or a prostate gland etc.

An important issue when performing biopsy sampling is that the needle collects a representative sample of the tissue. A typical prior art biopsy needle, for example the tru-cut^{®} needle, has a fixed sample cavity size; for example, 19 mm is a common length of a prior art sample cavity length. Still most of the times a biopsy is performed using prior art biopsy arrangements the cavity is only partially filled with tissue when the biopsy has been performed. That is, often the full length of tissue that was intended to be sampled has not been collected by the needle. This means that there is risk of missing an important part of the tissue and the result of the biopsy may consequently not be representative of the area sampled. Occasionally prior art biopsy arrangements such as the tru-cut^{®} needle fail to collect tissue at all.

Furthermore, sometimes the area of interest is longer than the length of the standard cavity of a prior art biopsy needle such as the tru-cut^{®} needle. One such example is in transperineal prostate biopsy. Today multiple biopsies in line are often required to sample the full length of the prostate.

Yet another problem with prior art biopsy needles such as the the tru-cut^{®} needle is that, from a point of view of an operator of the biopsy arrangement, training is required to remove the biopsy specimen from the biopsy cavity. Still with expertise there is a risk of fragmentation and subsequent quality reduction of the biopsy specimen using current technique.

Another important issue when performing biopsy sampling is, of course, to safeguard against accidental damage to a patient and to minimize the risks of infection.

### SUMMARY

In view of the above, an object of the proposed technology is to overcome drawbacks related to prior art biopsy arrangements.

According to a first aspect of the proposed technology, a biopsy needle arrangement is provided that comprises a needle sheath having proximal end and a distal end. The needle sheath constitutes an elongated tube having a sheath opening at the distal end of the needle sheath. The biopsy needle arrangement also comprises a needle having proximal end and a distal end and a length that is greater than the length of the needle sheath. The needle comprises an elongated shaft portion configured to fit inside and slide relative to the needle sheath, and a tip located at the distal end of the needle.

The biopsy needle arrangement further comprises an intermediary, or an intermediary shaft, having a proximal end and a distal end and a length that is greater than the length of the needle sheath. The intermediary comprises, or is composed by, an elongated shaft portion parallel with the needle and configured to fit inside the needle sheath and configured to slide relative to the needle sheath and slide relative to the needle.

In other words, such a biopsy needle arrangement comprises three main components: a needle, an intermediary and a needle sheath. When actuated, e.g. by being operatively connected to an actuator device, the needle sheath, the needle and the intermediary can move distally and/or proximally for obtaining a biopsy sample. Here, a movement distally is understood as a movement from a first position to a second position, the second position being more distal than the first position, and movement proximally is understood as a movement from a first position to a second position, the second position being more proximal than the first position.

The needle and the intermediary can slide along each other within the sheath and interact with each other and the sheath to create a cavity that becomes filled with tissue during a biopsy procedure. The relative position, in the distal/proximal direction of sliding during the biopsy procedure, of the needle and the intermediary will determine the length of the cavity and thereby determine the size of the tissue sample. With such an adaptive length of the biopsy cavity longer and continuous biopsies may be performed. This advantageously results in better pathology results with a minimum number of biopsies. A minimized number of biopsies also minimizes discomfort for a patient being subject to the biopsy procedure.

Furthermore, one explanation for the often only partial filling of the biopsy cavity when using prior art biopsy arrangements such as a the tru-cut^{®} needle is that the needle cavity is filled with air and thus not empty to start with. The air in the cavity needs to be evacuated in order for the cavity to being filled with tissue during the biopsy procedure. For prior art biopsy arrangements, such as a tru-cut^{®} biopsy arrangement, the air typically has nowhere to go. Such blocking by an air-filled cavity may inhibit the cavity from fully being filled with tissue. By the use of an intermediary as described herein, the biopsy cavity may be opened at the actual time of the biopsy procedure whereby this problem is eliminated. The result is a better filling of the cavity with longer and more representative biopsy samples.

The needle sheath may have a circular cross-section in a plane perpendicular to the longitudinal extension of the biopsy needle arrangement. The needle may have a first cross-sectional area, in a plane perpendicular to the longitudinal extension of the biopsy needle arrangement, and the intermediary may have a second cross-sectional area, in the plane perpendicular to the longitudinal extension of the biopsy needle arrangement.

The first cross-sectional area and the second cross-sectional area may have a respective constant size and shape between the proximal ends and the distal ends of the needle and the intermediary.

The needle and the intermediary may have complementary cross-sections that combine with each other and fit within the inner circumference of the sheath. This means that the shapes of the cross-sections are complementary and jointly form, or correspond to, the shape of the inner circumference of the sheath. The specific dimensions of the complementary cross-sections may be such that, within manufacturing tolerances, they can slide in relation to each other while at the same time minimizing any gap in relation to each other and in relation to the inner circumference of the sheath.

The first cross-sectional area may be any of a circular segment and a circular sector, and the second cross-sectional area may have a shape that is complementary to the first cross-sectional area. Alternatively, the second cross-sectional area may be any of a circular segment and a circular sector, and the first cross-sectional area may have a shape that is complementary to the second cross-sectional area.

The needle sheath may have a rectangular cross-section in a plane perpendicular to the longitudinal extension of the biopsy needle arrangement. The needle may have a first cross-sectional area, in a plane perpendicular to the longitudinal extension of the biopsy needle arrangement, and the intermediary may have a second cross-sectional area, in the plane perpendicular to the longitudinal extension of the biopsy needle arrangement. The first cross-sectional area may be a rectangle, and the second cross-sectional area may be a rectangle that is complementary to the first cross-sectional area.

Alternatively, other geometries in terms of cross-sections may be used. For example, oval or polygon shapes such as triangular cross-sections are also feasible.

The ratio between the size of the first cross-sectional area and the size of the second cross-sectional area may be in the interval 0.25 to 2, or 0.25 to 1.

That is, the cross-section of the needle and the cross-section of the intermediary may have relative sizes in a wide range. A relatively voluminous cavity, allowing for a relatively large tissue sample, can be obtained by a needle having a cross-sectional area that is smaller than the complementary cross-sectional area of the intermediary. Such a configuration may be used when the needle is made of a strong material such as Kevlar and/or in a use case where needle, intermediary and sheath provide mutual support as will be exemplified herein. A less voluminous cavity can be obtained by a needle having a cross-sectional area that is larger than the complementary cross-sectional area of the intermediary. Such a configuration may be used when the needle is made of a less strong material. A ratio of around 1 or 2/3 between the cross-sectional areas of the needle and the intermediary is a choice allowing for a voluminous cavity while at the same time ensuring a sturdy needle. Moreover, in terms of simplifying manufacturing of the needle and the intermediary (as well as the tubular sheath), a circular geometry may be selected and a ratio between the sizes of the first and second cross-sectional areas around 1.

The distal end of the intermediary may be configured with, or form, a front surface that is slanted relative to the longitudinal extension of the biopsy needle arrangement, or a surface in a plane that has a normal direction that is not parallel with the longitudinal extension of the biopsy needle arrangement.

In other words, the intermediary may be provided with a slanted distal end surface that may act as a "plough", or that is configured to lift the tissue of the tissue of the biopsy sample, when removing an obtained biopsy sample from the cavity. By moving such an intermediary in the distal direction, the tissue of the biopsy sample may be gently pushed out of the cavity and thereby maximizing the chance of retaining the sample in one piece. The actual direction of the normal direction, and consequently the slant of the distal end surface, may be selected such that the tissue of the biopsy sample dislodges from the cavity in a desired direction when being "ploughed" by the distal end of the intermediary.

The tip portion may have a transverse extension, or width, that is less than a transverse extension, or width, of the sheath opening.

In other words, the three-component configuration of sheath, needle and intermediary may have a configuration at the tip portion that is similar to prior art biopsy needle arrangements. For example, a prior art tru-cut^{®} needle arrangement may be improved by being configured with a needle and intermediary as disclosed herein.

The tip portion may have a transverse extension, or width, that is greater than a transverse extension, or width, of the sheath opening. This differs from prior art tru-cut^{®} biopsy needle arrangements in that they are closed-tip configurations whereas prior art tru-cut^{®} biopsy needle arrangements are open-tip configurations. An advantage of closed-tip configurations is that they enable a minimization of collection of bacteria during a biopsy procedure. For example, by providing the tip portion with a biassing force in relation to the sheath during insertion, the tip portion can be kept tight against the sheath with a resulting advantage in that collection of bacteria can be minimized.

The biopsy needle arrangement may comprise a needle connector configured to attach the needle to an actuator device, a sheath connector configured to attach the needle sheath to the actuator device and an intermediary connector configured to attach the intermediary to the actuator device.

The needle connector may be configured to disengage the needle from the actuator device when the needle is subjected to a force in the sliding direction, which exceeds a threshold force. Additionally or alternatively, the sheath connector may be configured to disengage the needle sheath from the actuator device when the needle sheath is subjected to a force in a direction opposite the sliding direction, which exceeds a threshold force.

The needle connector may comprise a first needle connector part configured to be attached to the actuator device, and a second needle connector part attached to the needle. When the needle disengages from the actuator device, the first needle connector part disengages from the second needle connector part and the second needle connector part moves distally in the sliding direction relative to the first needle connector part.

The sheath connector may comprise a first sheath connector part configured to be attached to the actuator device, and a second sheath connector part attached to the needle sheath. When the needle sheath disengages from the actuator device, the first sheath connector part disengages from the second sheath connector part and the first sheath connector part moves distally in the sliding direction relative to the second sheath connector part. The needle connector and/or the sheath connector may be any of a click-fit configuration of the respective first and second connector parts, a break-apart configuration of the respective first and second connector parts, and a friction fit configuration of the respective first and second connector parts.

This has the advantageous effect of preventing the biopsy needle arrangement from being damaged as a consequence of an unwanted collision between the sheath and the needle tip during a step where the sheath is moved distally to cut the biopsy sample.

In a second aspect of the proposed technology a method of operating a biopsy needle arrangement according to the above aspect is provided. This means that the biopsy needle arrangement comprises a needle, a needle sheath and an intermediary as defined above.

The biopsy needle arrangement may have an initial closed state and a final closed state. In the initial closed state, the distal end of the sheath may be located at the distal end, or the tip, of the needle. Additionally, the distal end of the intermediary may be located at the distal end, or the tip, of the needle. In the final closed state, the distal end of the sheath may be located at the distal end, or the tip, of the needle. Additionally, the distal end of the intermediary may be separated from the distal end, or the tip, of the needle. This, way the cavity formed between the needle, or the tip of the needle, and the intermediary, is enclosed by the sheath in the final closed state.

Starting from the initial closed state, the method may comprise: moving the needle distally, or forward, relative to the intermediary and the sheath. Here it is understood that the intermediary and the sheath is at rest relative to the surroundings. The method may further comprise: moving the sheath distally, or forward, relative to the needle and the intermediary so as to set the biopsy needle arrangement in the final closed state.

Worded differently, the method may comprise a sequence, starting from an initial closed state, of moving the needle distally while holding the intermediary stationary, thereby setting the needle arrangement in an open state and creating a cavity between the needle and the intermediary. This may then be followed by moving the sheath distally, thereby setting the needle arrangement in a final closed state

Alternatively, starting from the initial closed state, the method may comprise: moving the needle distally, or forward, relative to the sheath and moving the intermediary proximally, or rearward, relative to the sheath. Here it is understood that the sheath is at rest relative to the surroundings. The method may further comprise: moving the sheath distally, or forward, relative to the needle and the intermediary so as to set the biopsy needle arrangement in the final closed state.

Worded differently, the method may comprise a sequence, starting from the initial closed state, of moving the needle distally while moving the intermediary proximally, thereby setting the needle arrangement in an open state and creating a cavity between the needle and the intermediary. This may then be followed by moving the sheath distally, thereby setting the needle arrangement in a final closed state.

Alternatively, starting from the initial closed state, the method may comprise: moving the needle, the sheath and the intermediary distally, or forward, then moving the sheath and the intermediary proximally, or rearward, relative to the needle. Here it is understood that the needle is at rest relative to the surroundings. The method may further comprise: moving the sheath distally, or forward, relative to the needle and the intermediary so as to set the biopsy needle arrangement in the final closed state.

Worded differently, the method may comprise a sequence, starting from the initial closed state, moving the needle arrangement distally while in the closed state, then moving the sheath and the intermediary proximally, thereby setting the needle arrangement in an open state and creating a cavity between the needle and the intermediary. This may then be followed by moving the sheath distally, thereby setting the needle arrangement in a final closed state.

In a third aspect of the proposed technology, a biopsy system is provided comprising a biopsy needle arrangement according to the first aspect of the proposed technology. The biopsy system further comprises an actuator configured to operate the biopsy needle arrangement according to the method of the second aspect of the proposed technology.

Worded differently, the biopsy system may comprise a biopsy needle arrangement that comprises a needle, a needle sheath and an intermediary as summarized above, and an actuator. In such a system the actuator may be configured to cause a sequence, starting from an initial closed state, of moving the needle distally while holding the intermediary stationary, thereby setting the needle arrangement in an open state and creating a cavity between the needle and the intermediary. The actuator may further be configured to cause the sheath to move distally, thereby setting the needle arrangement in a closed state.

Alternatively, the actuator may be configured to cause a sequence, starting from the initial closed state, of moving the needle distally while moving the intermediary proximally, thereby setting the needle arrangement in an open state and creating a cavity between the needle and the intermediary. The actuator may further be configured to cause the sheath to move distally, thereby setting the needle arrangement in a closed state.

Alternatively, the actuator may be configured to cause a sequence, starting from the initial closed state, of moving the needle arrangement distally while in the closed state, then moving the sheath and the intermediary proximally, thereby setting the needle arrangement in an open state and creating a cavity between the needle and the intermediary. The actuator may further be configured to cause the sheath to move distally, thereby setting the needle arrangement in a closed state.

### BRIEF DESCRIPTION OF THE DRAWINGS

Figure 1a is a schematically illustrated side view of components of a biopsy needle arrangement,
figure 1b is a schematically illustrated side view of the biopsy needle arrangement in figure 1a,
figure 1c is a schematically illustrated side view of the biopsy needle arrangement in figure 1a,
figures 2a-d are schematically illustrated cross-sectional views of a biopsy needle arrangement,
figure 3 is a schematically illustrated cross-sectional view of a biopsy needle arrangement, figure 4 is a schematically illustrated cross-sectional view of a biopsy needle arrangement, figure 5 is a schematically illustrated side view of a detail of an intermediary and a needle in a biopsy needle arrangement,
figures 6a-b are schematically illustrated side views of a biopsy needle arrangement, figure 7 is a schematically illustrated side view of a biopsy needle arrangement,
figure 8 is a schematically illustrated side view of a biopsy needle arrangement with needle, intermediary and sheath connectors,
figures 9a-c schematically illustrate a first variant of a biopsy procedure,
figures 10a-c schematically illustrate a second variant of a biopsy procedure, and
figures 11a-d schematically illustrate a third and a fourth variant of a biopsy procedure.

### DETAILED DESCRIPTION

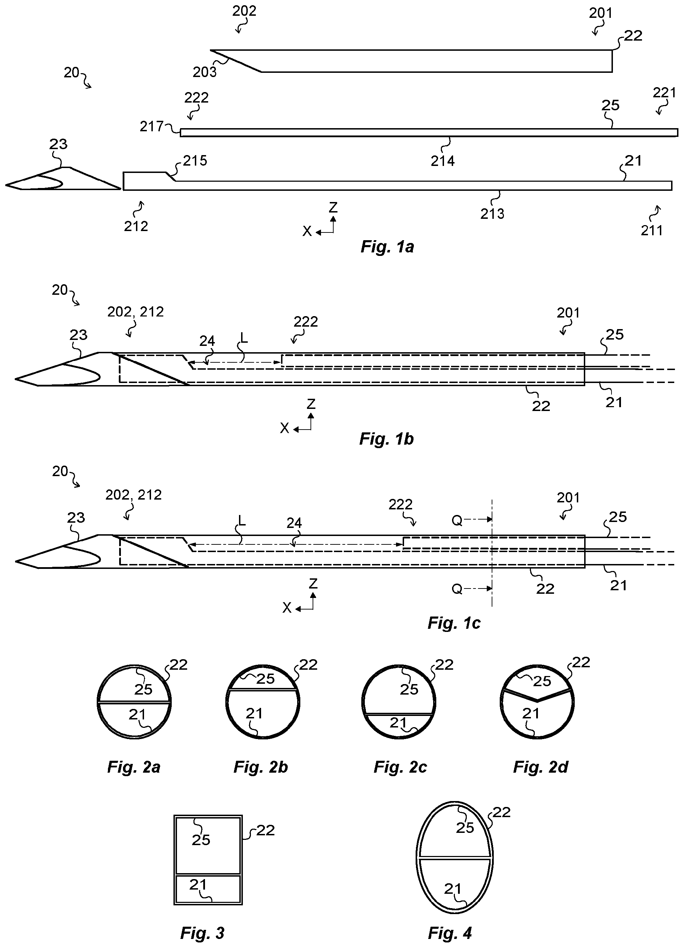

**Figures 1a****-c** illustrate a biopsy needle arrangement 20 that comprises a needle sheath 22 having proximal end 201 and a distal end 202. The needle sheath 22 constitutes an elongated tube having a sheath opening 203 at the distal end 202 of the needle sheath 22. The biopsy needle arrangement 20 also comprises a needle 21 having proximal end 211 and a distal end 212 and a length that is greater than the length of the needle sheath 22. The needle 21 comprises an elongated shaft portion 213 configured to fit inside and slide relative to the needle sheath 22, and a tip portion 23 located at the distal end 212 of the needle 21.

The biopsy needle arrangement 20 further comprises an intermediary 25, or an intermediary shaft, having a proximal end 221 and a distal end 222 and a length that is greater than the length of the needle sheath 22. The intermediary 25 comprises an elongated shaft portion 214 parallel with the needle 21 and configured to fit inside the needle sheath 22 and configured to slide relative to the needle sheath 22 and slide relative to the needle 21.

As will be illustrated in some more detail below, the biopsy needle arrangement 20 may be operatively connected to an actuator device for moving any of the needle sheath 22, the needle 21 and the intermediary 25 distally and/or proximally for obtaining a biopsy sample. The needle 21 and the intermediary 25 can slide along each other within the sheath 22 and interact with each other and the sheath 22 to create a cavity 24 between the distal end 222 of the intermediary and the distal end 212 of the needle 21 having a length L that becomes filled with tissue during a biopsy procedure. The length L of the cavity 24 may be adjusted to a desired value, as illustrated in figures 1b and 1c, by a movement of the intermediary 25 in the proximal and distal direction prior to or during a biopsy sampling procedure.

However, before describing such variants of how the biopsy needle arrangement 20 may be utilized in biopsy sampling procedures, some further illustrating details that may characterize the biopsy needle arrangement 20 will be discussed.

As indicated in figure 1a, the tip portion 23 of the needle 21 may at an initial manufacturing stage be an individual part that during manufacturing becomes a fixed part of the needle 21. Fixing of the tip portion 23 to the elongated shaft portion 213 of the needle 21 may entail, e.g., welding, friction welding or gluing the tip portion 23 to the distal end 212 of the needle 21. As illustrated in figure 1a, the distal end 212 of the needle 21 comprises a surface 215 that defines a distal end of the cavity 24. The surface 215 at the distal end 212 of the needle 21 is defined by a plane that is non-perpendicular to the direction X of the longitudinal extension of the needle arrangement 20. That is, the plane of the surface 215 is distally slanted away from the cavity 24, one reason for which being that the slanted surface 215 allows the distal end 202 of the sheath 22 to slide on the surface 215 during a closing of the needle arrangement 20 in case the sheath 22 and the tip portion 23 are not sufficiently aligned in the Z-direction during the closing.

As the skilled person will realize, the tip portion 23 of the needle 21 and the needle 21 may, instead of having been manufactured as individual parts that are fixed to each other as exemplified above, having been manufactured as one single unit. In the following description of embodiments, it is to be noted that it is irrelevant whether or not the tip portion 23 of the needle 21 and the needle 21 have initially been separate parts. It is also to be noted that cutting parts of the tip portion 23 may be configured in various ways, the description of which is outside the scope of the present disclosure.

As illustrated in **figures 2a****-d,** the needle sheath 22 may have a circular cross-section in a plane Q perpendicular to the longitudinal extension of the biopsy needle arrangement 20. The longitudinal extension is indicated by the direction X in figures 1a-c. The needle 21 has a first cross-sectional area An, in a plane Q perpendicular to the longitudinal extension of the biopsy needle arrangement 20, and the intermediary 25 has a second cross-sectional area Ai, in the plane Q perpendicular to the longitudinal extension of the biopsy needle arrangement 20.

The first cross-sectional area An may be any of a circular segment and a circular sector, and the second cross-sectional area Ai may have a shape that is complementary to the first cross-sectional area Ai. Alternatively, the second cross-sectional area Ai may be any of a circular segment and a circular sector, and the first cross-sectional area An may have a shape that is complementary to the second cross-sectional area Ai.

Figure 2a illustrates a configuration where the cross-sectional area An of the needle 21 is a semi-circle and the cross-sectional area Ai of the intermediary 25 is a complementary semi-circle. A ratio between the size of the cross-sectional area An of the needle 21 and the size of the cross-sectional area Ai of the intermediary 25 is about 1.

Figure 2b illustrates a configuration where the cross-sectional area An of the needle 21 is a circular segment and the cross-sectional area Ai of the intermediary 25 has a complementary shape where a ratio between the size of the cross-sectional area An of the needle 21 and the size of the cross-sectional area Ai of the intermediary 25 is about 2.

Figure 2c illustrates a configuration where the cross-sectional area An of the needle 21 is a circular segment and the cross-sectional area Ai of the intermediary 25 has a complementary shape where a ratio between the size of the cross-sectional area An of the needle 21 and the size of the cross-sectional area Ai of the intermediary 25 is about 0,5.

Figure 2d illustrates a configuration where the cross-sectional area An of the needle 21 is a circular sector and the cross-sectional area Ai of the intermediary 25 has a complementary shape where a ratio between the size of the cross-sectional area An of the needle 21 and the size of the cross-sectional area Ai of the intermediary 25 is about 1,5.

As illustrated in **figure 3****,** the needle sheath 22 may have a rectangular cross-section in a plane Q perpendicular to the longitudinal extension of the biopsy needle arrangement 20. The longitudinal extension is indicated by the direction X in figures 1a-c. The needle 21 has a first cross-sectional area An, in a plane Q perpendicular to the longitudinal extension of the biopsy needle arrangement 20, and the intermediary 25 has a second cross-sectional area Ai, in the plane Q perpendicular to the longitudinal extension of the biopsy needle arrangement 20. Here, the first cross-sectional area An is a rectangle, and the second cross-sectional area Ai is a rectangle that is complementary to the first cross-sectional area Ai.

Figure 3 illustrates a configuration where the cross-sectional area An of the needle 21 is a rectangle and the cross-sectional area Ai of the intermediary 25 has a complementary shape where a ratio between the size of the cross-sectional area An of the needle 21 and the size of the cross-sectional area Ai of the intermediary 25 is about 0,5.

In order to illustrate that the needle 21 and the intermediary 25 may have other cross-sectional shapes, **figure 4** is one such example where the cross-sectional area An of the needle 21 is a semi-oval and the cross-sectional area Ai of the intermediary 25 is a complementary semi-oval. A ratio between the size of the cross-sectional area An of the needle 21 and the size of the cross-sectional area Ai of the intermediary 25 is about 1.

Figures 2a-d, figure 3 and figure 4 thus illustrate that the ratio between the size of the first cross-sectional area An and the size of the second cross-sectional area Ai may be in the interval 0.25 to 2.

The first cross-sectional area An and the second cross-sectional area Ai may have a respective constant size and shape in all planes, including the plane Q as exemplified above, perpendicular to the longitudinal extension of the biopsy needle arrangement 20 between the proximal ends 201, 221 and the distal ends 202, 222 of the needle 21 and the intermediary 25.

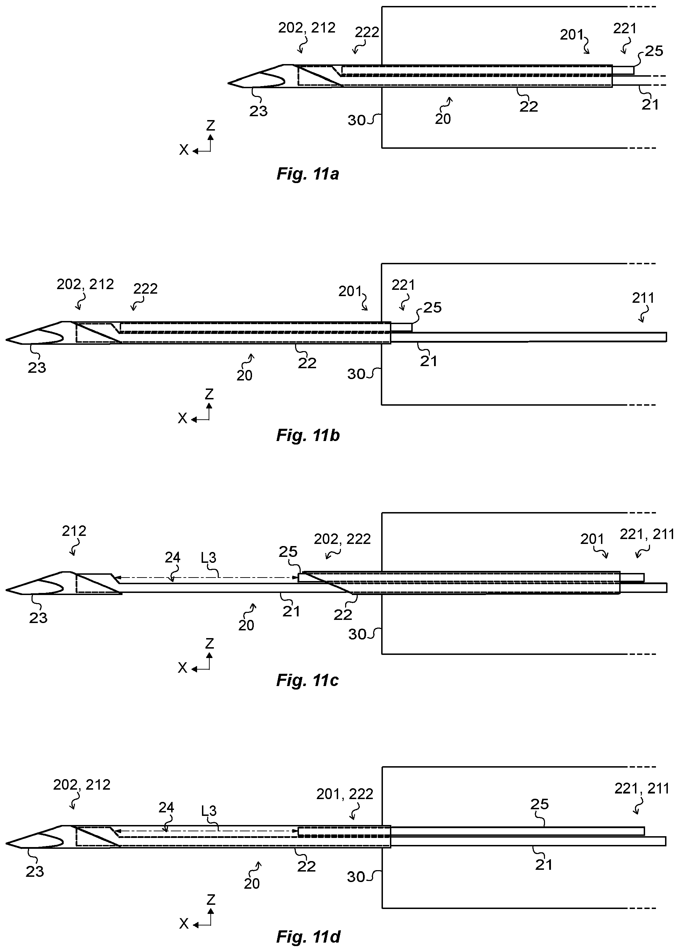

As mentioned above, the cross-section of the needle and the cross-section of the intermediary may have relative sizes in a wide range. A desire to configure the biopsy needle arrangement 20 with a cavity 24 of maximum volume, i.e. minimizing the first cross-sectional area An, may entail selecting a material for at least the needle 21 that is strong enough. For example, by selecting Kevlar as the material for the needle 21 will enable a design where the ratio between the size of the first cross-sectional area An and the size of the second cross-sectional area Ai may have a low value, e.g. any value as low as 0.5 or even as low as 0.25. However, as mentioned elsewhere, a low value for the ratio and a less strong material may be used for at least the needle 21 when using the needle arrangement 20 in a use case where the needle 21, the intermediary 25 and the sheath 22 provide mutual support when operated as will be exemplified below in connection with figure 11. Conversely, if at least the needle 21 is made of a less strong material, it may be desirable to select the ratio between the size of the first and second cross-sectional areas to be higher, for example any value from 0.5 to 1 or even 2.

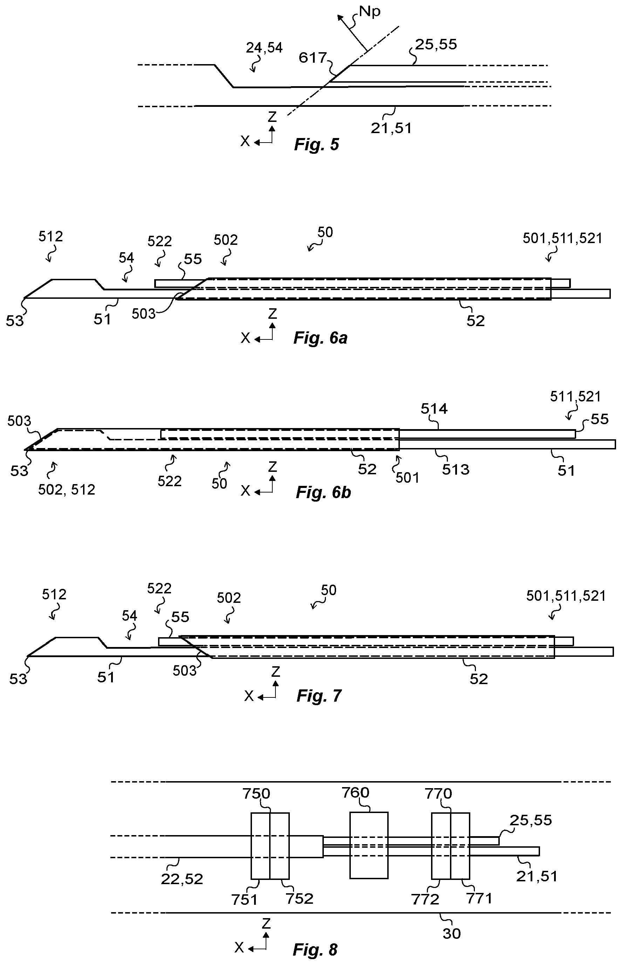

As exemplified in figures 1a-b, the distal end 222 of the intermediary 25 may have an end surface 217 that is perpendicular to the longitudinal extension of the biopsy needle arrangement 20. For example, the end surface 217 of the intermediary 25 may be defined by a plane that has a normal direction along the direction X as indicated in figures 1a-c. However, as illustrated in **figure 5****,** the distal end 222 of the intermediary 25 may configured with a surface 617 in a plane that has a normal direction Np that is not parallel with the longitudinal extension of the biopsy needle arrangement 20. Although figure 5 illustrates an example where the normal direction Np of the plane containing the surface 617, i.e. the end surface, lies in a plane defined by the X and Z directions, the normal direction Np may be directed in other directions. For example, the end surface 617 may be defined by a plane having a normal direction that is not in the plane defined by the X and Z directions, i.e. a direction that is partly in the Z direction and partly in a direction perpendicular to the X and Z directions. In other words, as discussed above, the slanted distal end surface 617 may be seen as a "plough" surface.

**Figures 6a****-b** illustrate an embodiment of a biopsy needle arrangement 50 comprising a needle sheath 52 having proximal end 501 and a distal end 502, the needle sheath 52 constituting an elongated tube having a sheath opening 503 at the distal end 502 of the needle sheath 52. The biopsy needle arrangement 50 also comprises a needle 51 having proximal end 511 and a distal end 512 and a length that is greater than the length of the needle sheath 52, the needle 51 comprising an elongated shaft portion 513 configured to fit inside and slide relative to the needle sheath 52, and a tip portion 53 located at the distal end 512 of the needle 51.

The biopsy needle arrangement 50 further comprises an intermediary 55 having proximal end 521 and a distal end 522 and a length that is greater than the length of the needle sheath 52, the intermediary 55 comprising an elongated shaft portion 514 parallel with the needle 51 and configured to fit inside the needle sheath 52 and configured to slide relative to the needle sheath 52 and slide relative to the needle 51.

In the embodiment of the biopsy needle arrangement 50 illustrated in figures 6a-b, the tip portion 53 has a transverse extension, or width, that is less than a transverse extension, or width, of the sheath opening 503. The needle arrangement 50 differs from the needle arrangement 20 illustrated in figures 1a-c in that in the needle arrangement 20 the tip portion 23 has a transverse extension, or width, that is greater than a transverse extension, or width, of the sheath opening 203.

The features relating to cross-sectional areas of the needle 21, sheath 22 and intermediary 25 described above in connection with figures 2a are applicable also for the biopsy needle arrangement 50 comprising the needle 51, sheath 52 and intermediary 55. Furthermore, although not illustrated in figures 6a-b, the intermediary 55 may be configured with a distal end surface in the same way as the examples of end surfaces 217, 617 discussed above in connection with figures 1a-c and figure 5.

The biopsy needle arrangement 50 illustrated in figures 6a-b illustrates that a "three component" approach with a needle 51, a sheath 52 and an intermediary 55 may be applied to a prior art biopsy needle arrangement such as a tru-cut^{®} needle arrangement. That is, it is applicable also where the tip portion 53 fits inside the sheath 52 when the sheath 52 has been moved distally and cut a tissue sample filling the cavity 54 between the distal end 522 of the intermediary 55 and the distal end 512 of the needle 51.

The sheath opening 503 at the distal end 502 of the sheath 52 illustrated in figures 6a-b lies in a plane that has a normal direction that is not parallel with the longitudinal (i.e. along the X direction) extension of the biopsy needle arrangement 50. In figures 6a-b, such a normal direction has a positive value both in the X direction and in the Z direction. **Figure 7** illustrates an example where the sheath opening 503 at the distal end 502 of the sheath 52 lies in a plane that has a normal direction that also is not parallel with the longitudinal extension of the biopsy needle arrangement 50. However, in contrast to the direction in figures 6a-b, the normal direction of the plane of the sheath opening 503 illustrated in figure 7 has a positive value in the X direction but a negative value in the Z direction.

Although not illustrated, another alternative is that the sheath opening 503 at the distal end 502 of the sheath 52 lies in a plane that has a normal direction that is parallel with the longitudinal extension of the biopsy needle arrangement 50. In fact, with reference also to the sheath 22 illustrated elsewhere herein, also the sheath opening 203 at the distal end 202 of the sheath 22 may lie in a plane that has a normal direction that is parallel with the longitudinal extension of the biopsy needle arrangement 20

Turning now to **figure 8****,** and with continued reference to figures 1a-c, figures 6a-b and figure 7, the biopsy needle arrangement 20, 50 may further comprise a needle connector 770 configured to attach the needle 21, 51 to an actuator device 30, a sheath connector 750 configured to attach the needle sheath 22, 52 to the actuator device 30 and an intermediary connector 760 configured to attach the intermediary 25, 55 to the actuator device 30. As indicated above, such an actuator device 30 may be configured such that it is capable of moving any of the needle sheath 22, 52, the needle 21, 51 and the intermediary 25, 55 distally and/or proximally for obtaining a biopsy sample. Details regarding such configuration of the actuator device 30 is outside the scope of the present disclosure.

The needle connector 770 may be configured to disengage the needle 21, 51 from the actuator device 30 when the needle 21, 51 is subjected to a force in the sliding direction X, which exceeds a threshold force, and/or the sheath connector 750 may be configured to disengage the needle sheath 22, 52 from the actuator device 30 when the needle sheath 22, 52 is subjected to a force in a direction -x opposite the sliding direction x, which exceeds a threshold force.

For example, the needle connector 770 may comprise a first needle connector part 771 configured to be attached to the actuator device 30, and a second needle connector part 772 attached to the needle 21, 51, and when the needle 21, 51 disengages from the actuator device 30, the first needle connector part 771 disengages from the second needle connector part 772 and the second needle connector part 772 moves distally in the sliding direction x relative to the first needle connector part 771. Similarly, for example, the sheath connector 750 may comprise a first sheath connector part 751 configured to be attached to the actuator device 30, and a second sheath connector part 752 attached to the needle sheath 22, 52, and when the needle sheath 22, 52 disengages from the actuator device 30, the first sheath connector part 751 disengages from the second sheath connector part 752 and the first sheath connector part 751 moves distally in the sliding direction x relative to the second sheath connector part 752. In such embodiments, the needle connector 370 and/or the sheath connector 350 may be any of a click-fit configuration of the respective first and second connector parts 771, 772, 751, 752, a break-apart configuration of the respective first and second connector parts 771, 772, 751, 752, and a friction fit configuration of the respective first and second connector parts 771, 772, 751, 752.

By configuring the biopsy needle arrangement 20, 50 according to such embodiments it is possible to protect the biopsy needle arrangement 20, 50 from being damaged as a consequence of, e.g., an unwanted collision between the sheath 22, 52 and the needle tip 23, 53 during a step where the sheath 22, 52 is moved distally to cut the biopsy sample. Needless to say, such a collision is less likely to occur when the tip portion 53 fits within the sheath 52 as exemplified in figures 6a-b and figure 7 than in a case where the tip portion 23 has a transverse extension, or width, that is greater than a transverse extension, or width, of the sheath opening 203. Nevertheless, protection from damage due to a collision between the sheath 52 and the tip portion 53, even though the tip portion 53 is configured to fit within the sheath 52, may be relevant for example in case the tip portion 53 becomes bent during an initial insertion into tissue to be subject to the biopsy sampling.

As already discussed at some length, the biopsy needle arrangement 20, 50 may be operated by, e.g., during a biopsy procedure by an operator for obtaining a biopsy tissue sample from a body part of a patient. Depending on various factors, such as tissue properties and desired length of biopsy , the components of the biopsy needle arrangement 20, 50 may be controlled via an actuator 30 to move in the distal direction and in the proximal direction during the biopsy procedure.

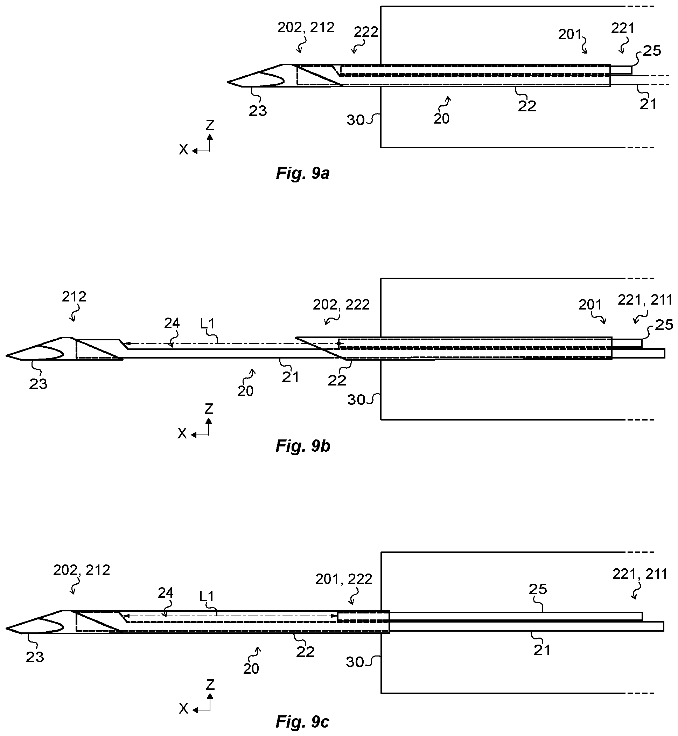

Turning now to **figures 9a****-c,** **figures 10a****-c** and **figures 11a****-d,** these figures illustrate variants of a biopsy procedure where a three-component needle arrangement is advantageously utilized to obtain biopsy samples. The biopsy needle arrangement 20 described in connection with figures 1a-c is used for these illustrations. However, it is to be noted that the biopsy needle arrangement 50 described in connection with figures 6a-b and figure 7 may also be used. The variants of a biopsy procedure illustrated in figures 9a-c, figures 10a-c and figures 11a-d include a symbolic actuator 30. The actuator 30 may be any kind of actuator, automatic or semi-automatic, that is appropriately configured to move the components of the needle arrangement 20 as described. In fact, the needle arrangement 20 may also be operated more or less manually without the use of an actuator.

A first variant is illustrated in figures 9a-c and **figure 9a** illustrates an initial relative position of the components of the biopsy needle arrangement 20 where the biopsy needle arrangement 20 is in a closed state and where the tip portion 23 is located adjacent an area of tissue that is to be sampled.

**Figure 9b** illustrates a completed first step wherein the needle 21 has been caused by the actuator 30 to move distally, i.e. in the direction X that defines the longitudinal extension of the biopsy needle arrangement 20. The biopsy needle arrangement 20 has now obtained an open state where the cavity 24 between the distal end 222 of the intermediary and the distal end 212 of the needle 21 has been created with a length L1 and filled with tissue. The length L1 may be determined by an adjustment of the initial position in the X direction of the intermediary 25 in relation to the distal end 212 of the needle 21.

**Figure 9c** illustrates a completed second step wherein the sheath 22 has been caused by the actuator 30 to move distally, i.e. in the direction X that defines the longitudinal extension of the biopsy needle arrangement 20. During the movement of the sheath 22, the tissue within the cavity 24 has been cut and the biopsy needle arrangement 20 has now obtained a closed state and the tissue in the cavity 24 is enclosed by the cavity 24 inside the tubular sheath 22.

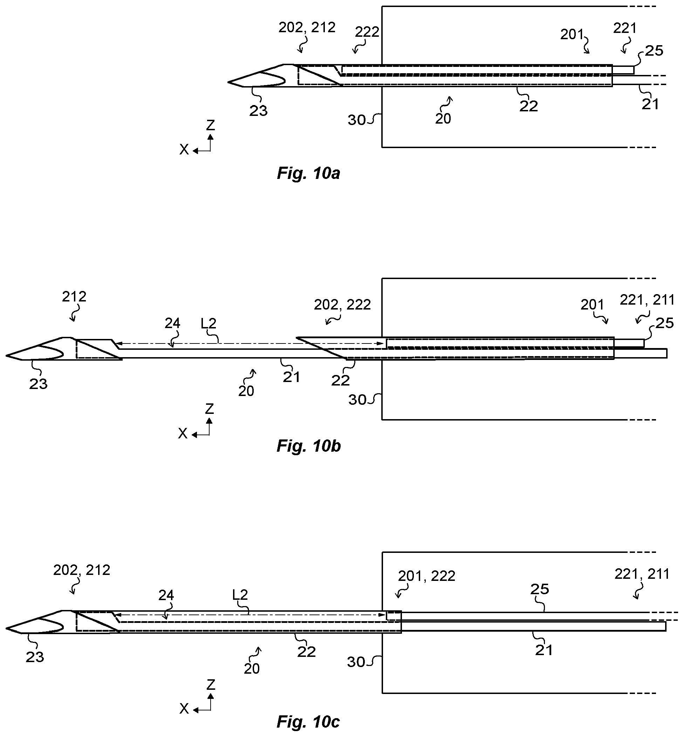

A second variant is illustrated in figures 10a-c and **figure 10a** illustrates an initial relative position of the components of the biopsy needle arrangement 20 where the biopsy needle arrangement 20 is in a closed state and where the tip portion 23 is located adjacent an area of tissue that is to be sampled.

**Figure 10b** illustrates a completed first step wherein the needle 21 has been caused by the actuator 30 to move distally, i.e. in the direction X, and the intermediary 25 has been caused by the actuator 30 to move proximally i.e. in the direction -X. The biopsy needle arrangement 20 has now obtained an open state where the cavity 24 between the distal end 222 of the intermediary and the distal end 212 of the needle 21 has been created with a length L2 and filled with tissue. The length L2 may be determined by an adjustment of the initial position in the X direction of the intermediary 25 in relation to the distal end 212 of the needle 21 and by an adjustment of a distance by which the intermediary 25 is moved proximally during this first step. An advantage of moving the intermediary 25 proximally during this first step is that it creates a "vacuum effect" that attracts tissue into the cavity 24 that is created during this first step. This may also allow for better tissue filling by suction of fluids from the biopsy area that would otherwise have obstructed the chamber from filling with tissue.

**Figure 10c** illustrates a completed second step wherein the sheath 22 has been caused by the actuator 30 to move distally, i.e. in the direction X that defines the longitudinal extension of the biopsy needle arrangement 20. During the movement of the sheath 22, the tissue within the cavity 24 has been cut and the biopsy needle arrangement 20 has now obtained a closed state and the tissue in the cavity 24 is enclosed by the cavity 24 inside the tubular sheath 22.

A third variant is illustrated in figures 11a-d and **figure 11a** illustrates an initial relative position of the components of the biopsy needle arrangement 20 where the biopsy needle arrangement 20 is in a closed state and where the tip portion 23 is located adjacent an area of tissue that is to be sampled.

**Figure 11b** illustrates a completed first step wherein the needle 21, the intermediary 25 and the sheath 22 have been caused by the actuator 30 to move distally, i.e. in the direction X. That is, the biopsy needle arrangement 20 remains in the closed state during this first step. An effect of this is that the needle 21, intermediary 25 and sheath 22 provide mutual support for each other during this step of insertion into tissue. The sequence thereby allows for using a needle arrangement where the ratio between the size of the cross-sectional area of the needle and the size of the cross-sectional area of the intermediary has a relatively low value, i.e. where the needle is "thin" and the volume of the cavity becomes relatively voluminous. Also, by moving the needle arrangement 20 while in the closed state, it is very stable and it is thus possible to achieve minimal spatial deviation from an intended target located deep within a body. Furthermore, having positioned the needle arrangement 20 stably at the target in this closed state, the following second and third steps may be performed such that a maximum cavity length can be used.

A variation of such a first step is that, instead of being caused by the actuator 30, the needle 21, the intermediary 25 and the sheath 22 are moved distally by hand without assistance of the actuator 30. Such a variation of the first step may be advantageous in a scenario where it is extremely important that the biopsy needle arrangement 20 is exactly positioned, e.g. in relation to sensitive areas such as blood vessels and nerves.

**Figure 11c** illustrates a completed second step wherein the intermediary 25 and the sheath 22 have been caused by the actuator 30 to move proximally, i.e. in the direction - X, while the needle 21 remain at the position obtained during the first step. The biopsy needle arrangement 20 has now obtained an open state where the cavity 24 between the distal end 222 of the intermediary and the distal end 212 of the needle 21 has been created with a length L3 and filled with tissue. The length L3 may be determined by an adjustment of a distance by which the intermediary 25 is moved proximally during this second step. As an option, the length of the cavity 24 may be increased by moving the intermediary 25 proximally. An advantage of moving the intermediary 25 proximally during this second step would then be a "vacuum effect" that attracts tissue into the cavity 24 that is created during this second step, as in the procedure variant illustrated above in connection with figures 10a-c.

**Figure 11d** illustrates a completed third step wherein the sheath 22 has been caused by the actuator 30 to move distally, i.e. in the direction X that defines the longitudinal extension of the biopsy needle arrangement 20. During the movement of the sheath 22, the tissue within the cavity 24 has been cut and the biopsy needle arrangement 20 has now obtained a closed state and the tissue in the cavity 24 is enclosed by the cavity 24 inside the tubular sheath 22.

Subsequent to the steps of closing the biopsy needle arrangement 20 as illustrated in figure 9c, figure 10c and figure 11d, the biopsy needle arrangement 20 may be withdrawn in the proximal direction, i.e. in the direction -X, whereupon the biopsy needle arrangement 20 may be set in the open state. The setting into the open state involves movement of the sheath 22 in the proximal direction and it may be caused by the actuator 30 or by manual action. The cavity 24 may then be emptied of tissue in a conventional way or emptied by a step of moving the intermediary 25 distally, manually or via the actuator 30, and thereby performing a "ploughing" action. Such a "ploughing" action may preferably be utilized using a biopsy needle arrangement 20 where the intermediary 25 is configured with a distal end surface 617 that is slanted, as described above in connection with figure 5. The sheath 22 may be caused to move forward together with the intermediary 25, thereby providing stability that ensures that "the plough" is in contact with the bottom of the cavity 24.

## Claims

1. A biopsy needle arrangement (20) comprising:
- a needle sheath (22) having proximal end (201) and a distal end (202), the needle sheath (22) constituting an elongated tube having a sheath opening (203) at the distal end (202) of the needle sheath (22),
- a needle (21) having proximal end (211) and a distal end (212) and a length that is greater than the length of the needle sheath (22), the needle (21) comprising an elongated shaft portion (213) configured to fit inside and slide relative to the needle sheath (22), and a tip portion (23) located at the distal end (212) of the needle (21),
- an intermediary (25) having proximal end (221) and a distal end (222) and a length that is greater than the length of the needle sheath (22), the intermediary (25) comprising an elongated shaft portion (214) parallel with the needle (21) and configured to fit inside the needle sheath (22) and configured to slide relative to the needle sheath (22) and slide relative to the needle (21).

2. The biopsy needle arrangement (20) of claim 1, wherein:
- the needle sheath (22) has a circular cross-section in a plane (Q) perpendicular to the longitudinal extension of the biopsy needle arrangement (20),
- the needle (21) has a first cross-sectional area (An), in a plane (Q) perpendicular to the longitudinal extension of the biopsy needle arrangement (20), and the intermediary (25) has a second cross-sectional area (Ai), in the plane (Q) perpendicular to the longitudinal extension of the biopsy needle arrangement (20), wherein:
- the first cross-sectional area (An) is any of a circular segment and a circular sector, and the second cross-sectional area (Ai) has a shape that is complementary to the first cross-sectional area (Ai), or wherein:
- the second cross-sectional area (Ai) is any of a circular segment and a circular sector, and the first cross-sectional area (An) has a shape that is complementary to the second cross-sectional area (Ai).

3. The biopsy needle arrangement (20) of claim 1, wherein:
- the needle sheath (22) has a rectangular cross-section in a plane (Q) perpendicular to the longitudinal extension of the biopsy needle arrangement (20),
- the needle (21) has a first cross-sectional area (An), in a plane (Q) perpendicular to the longitudinal extension of the biopsy needle arrangement (20), and the intermediary (25) has a second cross-sectional area (Ai), in the plane (Q) perpendicular to the longitudinal extension of the biopsy needle arrangement (20), wherein:
- the first cross-sectional area (An) is a rectangle, and the second cross-sectional area (Ai) is a rectangle that is complementary to the first cross-sectional area (Ai).

4. The biopsy needle arrangement (20) of any of claims 2 to 3, wherein:
- the first cross-sectional area (An) and the second cross-sectional area (Ai) have a respective constant size and shape between the proximal ends (201, 221) and the distal ends (202, 222) of the needle (21) and the intermediary (25).

5. The biopsy needle arrangement (20) of any of claims 2 to 4, wherein:
- the ratio between the size of the first cross-sectional area (An) and the size of the second cross-sectional area (Ai) is in the interval 0.25 to 2.

6. The biopsy needle arrangement (20) of any of claims 1 to 5, wherein:
- the distal end (222) of the intermediary (25) is configured with a surface (617) in a plane that has a normal direction (Np) that is not parallel with the longitudinal extension of the biopsy needle arrangement (20).

7. The biopsy needle arrangement (50) of any of claims 1 to 6, wherein the tip portion (53) has a transverse extension, or width, that is less than a transverse extension, or width, of the sheath opening (503).

8. The biopsy needle arrangement (20) of any of claims 1 to 6, wherein the tip portion (23) has a transverse extension, or width, that is greater than a transverse extension, or width, of the sheath opening (203).

9. The biopsy needle arrangement (20, 50) of any of claims 1 to 8, further comprising:
- a needle connector (770) configured to attach the needle (21, 51) to an actuator device (30), a sheath connector (750) configured to attach the needle sheath (22, 52) to the actuator device (30) and an intermediary connector (760) configured to attach the intermediary (25, 55) to the actuator device (30).

10. The biopsy needle arrangement (20) of claim 9, wherein:
- the needle connector (770) is configured to disengage the needle (21, 51) from the actuator device (30) when the needle (21, 51) is subjected to a force in the sliding direction (x), which exceeds a threshold force, and/or
- the sheath connector (750) is configured to disengage the needle sheath (22, 52) from the actuator device (30) when the needle sheath (22, 52) is subjected to a force in a direction (-x) opposite the sliding direction (x), which exceeds a threshold force.

11. The biopsy needle arrangement (20) of claim 10, wherein:
- the needle connector (770) comprises a first needle connector part (771) configured to be attached to the actuator device (30), and a second needle connector part (772) attached to the needle (21, 51), and
- when the needle (21, 51) disengages from the actuator device (30), the first needle connector part (771) disengages from the second needle connector part (772) and the second needle connector part (772) moves distally in the sliding direction (x) relative to the first needle connector part (771).

12. The biopsy needle arrangement (20) of any of claims 10 to 11, wherein:
- the sheath connector (750) comprises a first sheath connector part (751) configured to be attached to the actuator device (30), and a second sheath connector part (752) attached to the needle sheath (22, 52), and
- when the needle sheath (22, 52) disengages from the actuator device (30), the first sheath connector part (751) disengages from the second sheath connector part (752) and the first sheath connector part (751) moves distally in the sliding direction (x) relative to the second sheath connector part (752).

13. The biopsy needle arrangement (20) of any of claims 11 to 12, wherein the needle connector (370) and/or the sheath connector (350) is any of:
- a click-fit configuration of the respective first and second connector parts (771, 772, 751, 752),
- a break-apart configuration of the respective first and second connector parts (771, 772, 751, 752), and
- a friction fit configuration of the respective first and second connector parts (771, 772, 751, 752).
